# EUROPÄISCHE PATENTANMELDUNG

(11) **EP 2 366 328 A1**
(43) Veröffentlichungstag der Anmeldung: **21.09.2011**
(21) Anmeldenummer: 10002726.7
(22) Anmeldetag: 16.03.2010
(51) Int. Cl.: A61B 3/08, A61B 3/113

(54) **Differenzmessung der monokularen zur binokularen Augenstellung**

(71) Anmelder: Stuetz, Ignaz Alois, 4212 Neumarkt im Muehlkreis (AT)
(72) Erfinder: Stuetz, Ignaz Alois, 4212 Neumarkt im Muehlkreis (AT)
(74) Vertreter: Piermayr, Alexander

(57) **Zusammenfassung**

Der menschliche Organismus weist spezielle Steuerungsmechanismen auf, um binokular, im besten Fall belastungsfrei stereoskopisch, sehen zu können. Häufig sind diese kompensatorischen Systeme überbelastet und führen bei Betroffenen zu asthenopischen Beschwerden und zu eingeschränkter oder fehlender Stereopsis. Die Erfindung erlaubt die objektive Darstellung der motorischen Vorgänge, indem zunächst die monokulare Fixation rechts und links kalibriert wird. Sodann werden binokulare Sehaufgaben gestellt und die Abweichungen gegenüber der monokularen Fixation samt kompensatorischem Bewegungsverhalten gemessen, aufgezeichnet und auf einem Kontrollmonitor in Relation zum subjektiven Fixationsobjekt schematisch dargestellt. Parallel zu subjektive Prüf- und Optimierungsverfahren werden damit diese motorisch-kompensatorischen Vorgänge bei binokularen Sehaufgaben für die Professionisten sichtbar. Somit kann die gegebene Sehsituation der Probanden schneller analysiert und bei Bedarf zügig eine optimal entlastende Korrektion ermittelt werden. Abschließend können Prüfer und Proband die Qualität verschiedener (auch prismatischer) Korrektionen vergleichen, beurteilen und klare Entscheidungen fällen.

## Beschreibung

### 1. Das technische Gebiet:

Der Mensch besitzt wie die Primaten ein hervorragendes binokulares System. Fixieren beide Augen auf das selbe Objekt ermöglicht das "Stereosehen", indem hoch entwickelte, neuronale Verarbeitungszentren ein räumliches Bild generieren, das in Sekundenbruchteilen eine dreidimensionaleWahmehmung der Außenwelt mit präzisen Entfernungs- und Geschwindigkeitsrelationen zur Verfügung stellt.

Trotz enormer Kompensationsfähigkeit für kleinere anatomische Defizite entwickelt sich beim Kleinkind diese Stereopsis manchmal nicht oder nicht optimal. Im Erwachsenenalter nimmt die organische Ausgleichsfähigkeit und Belastbarkeit ab, sodass es in Kombination mit einem individuell sehr unterschiedlichen Fusionsaufwand zur Reduktion der Stereotiefe kommen kann. Um einen Entwicklungsrück- bzw. Reduktionsstand zu erfassen und den Fortschritt bzw. Erfolg von Maßnahmen und Korrektionen zu überprüfen ist also die Erhebung und Dokumentation dieses Teils des visuellen Systems erforderlich.

### 2. Stand der Technik:

Zur Ermittlung des Status und die Wirkung allfälliger Korrektionen werden verschiedene Verfahren eingesetzt. Zu den objektiven Verfahren gehört die "freie" Beobachtung und Abschätzung der Augenstellung bzw. -bewegung, während die Probanden abwechselnd monokular und binokular auf ein ruhendes Objekt blicken müssen. Bei Motilitätstests, werden binokular (eventuell auch monokular) die Folgebewegungen des Auges beachtet. Bei Belastungstestverfahren werden jene (nahen) Entfernungen und Prismenstärken ermittelt, bei denen die Fusion der Probanden zusammenbricht sowie bei welcher sie wiederhergestellt werden kann.

Seit Jahrzehnten wird über verschiedene technische Anordnungen die Augenstellung gegenüber dem Kopf bzw. einem Gerät sowie die Art der Augenbewegungen exakt vermessen. Eine gängige Anwendung ist z.B. der augengesteuerte Autofokus bei Fotoapparaten und Videokameras. Es ist bekannt, dass es mehrere verschiedene, völlig natürliche und so minimale Augenbewegungen gibt, dass sie mit freiem Auge von Beobachtern nicht wahrgenommen werden können. Vgl. Patent EP1300108 (B1)

Neuere Verfahren ermöglichen die exakte objektive Vermessung der Augenstellung und -bewegungen auch gegenüber Objekten im Raum, wobei der Kopf des Probanden frei beweglich bleibt, indem das darauf befestigte System gleichzeitig die Blickbewegung des Auges bzw. des Augenpaares gegenüber dem Messsystem sowie dessen Ausrichtung im Raum kontrolliert (vgl. Patent EP 0 898 930 A2, Patent EP1319361 (A1)). Diese Messungen werden an ruhenden oder bewegten Objekten, in verschiedenen Blickrichtungen etc. durchgeführt und dienen monokular der Bestimmung des zentralen und peripheren Schvermögens, dem Auffinden von (pathologischen) Motilitätsstörungen sowie binokular zur Fusionbreitenmessung.

Bei der Prüfung der monokularen Fixation wird zu ermitteln versucht, auf welches Areal der Netzhaut ein visuelles Objekt abgebildet wird. Bei Anwendung des direkten Ophthalmoskops werden die Probanden relativ stark geblendet, steht ein modernes SLO (Scanning-Laser-Ophthalmoskop) zur Verfügung kommt es zu keiner besonderen Blendung der Probanden. Mit dem SLO können verschiedene, helle, dunkle, bewegliche Fixationsmarken, auch Wörter und ganze Sätze sind verwendbar, eingeblendet werden, sodass Fixationsobjekte sehr eindeutig dem PRL (Preferred Retinal Locus) der Netzhaut zugeordnet werden können. Diese monokulare Untersuchung kann entspannt über längere Zeit durchgeführt und aufgezeichnet werden. Die zusätzliche Kombination des SLO mit der OCT (Optische-Cohärenz-Tomographie, vgl. Patent WO 2009/059400 A1) dient diagnostischen Zwecken.

Die MKH (Mess- und Korrektionsmethodik nach H.-J. Haase) unterscheidet sich von obigen durch die reine Subjektivität. Probanden beschreiben ihre Wahrnehmung von Objekten, wodurch auch sehr feine Stellungs- und Steuerungsanomalien, sogenannte "Fixationsdisparationen", analysiert werden können. Ziel ist die Ermittlung einer (prismatischen) Korrektion, die den Probanden ein hochwertig-binokulares, belastbares und beschwerdefreies Schen ermöglicht.

### 3. Beschreibung der Erfindung im Detail:

Gegenüber den beschriebenen gängigen Verfahren besteht die Erfindung in einer technischen Anordnung, die geringste Abweichungen zwischen der monokularen und der binokularen Augenstellung bzw. -bewegung in Relation zu einem visuellen Objekt erfasst. Voraussetzung ist, dass zur Simulation alltäglicher Sehaufgaben nicht nur die unterschiedlichen Körperhaltungen, Blickrichtungen und Entfernungen beim Sehen in die Ferne, beim Gehen, bei handwerklichen Verrichtungen und beim Lesen repräsentiert sind, sondern auch die jeweilige typische Lichtsituation hergestellt wird. Dann interessieren die Messergebnisse im status "sine correctione" zum Unterschied von denen mit insbesondere prismatischen Korrektionen sowie die Veränderungen über einen längeren Zeitraum mit und ohne Korrektion, mithin die Belastbarkeit des körpereigenen kompensatorischen Systems.

Zunächst benötigt man eine Anordnung zur exakten Vermessung der Augenstellung. Derartige sind bekannt und können am Kopf oder auch in der Nähe des Kopfes befestigt sein. Als nächster Schritt wird den Probanden ein Objekt präsentiert und monokular die Fixation für das rechte und linke Auge, während das andere jeweils abgedeckt ist, "kalibriert", das heißt, eine Zuordnung der Augenstellung zum Ort des Objektes (auf einem Präsentationsmonitor) hergestellt. Auch diese Verfahren sind bekannt und beispielsweise bei Foto- und Videokameras mit augengesteuertem Autofocus im Einsatz.

Neu ist nun, dass, nachdem mit Hilfe der Vorrichtung die Fixation des Probanden einzeln monokular eingemessen wurde, diese in Relation zum Sehobjekt auf einem Kontrollmonitor dargestellt wird. Auf diesem Monitor wird für den Prüfer das Fixationsobjekt überlagert von einem Symbol für die Augenstellung abgebildet. Bei der Erfindung werden - zunächst noch unter monokularen Bedingungen, die bekannten Augenbewegungen, wie etwa des natürlichen minimalen Augenzitterns, der Drifts und des Rückholsprungs, diese Phänomene in Relation zum Sehobjekt dargestellt, zusätzlich exakt vermessen und aufgezeichnet als Winkel in Größe, Richtung und Veränderungsgeschwindigkeit.

Zentraler Punkt der Erfindung ist, dass diese exakte objektive Messung nun unter binokularen Bedingungen durchgeführt wird, dass also der Proband mit beiden Augen gleichzeitig auf das Objekt blickt. Am Kontrollmonitor wird die Blickrichtung für das rechte und linke Auge mit Symbolen gekennzeichnet, gleichzeitig die zugehörigen Winkel vom System gemessen und aufgezeichnet. Dabei interessiert vor allem, ob überhaupt und welches Auge eine Führungsrolle (sogenannte Dominanz oder Prävalenz) übernimmt und welches Auge als Begleitauge fungiert, in welchen Zeiträumen wie große Winkelabweichungen festzustellen sind etc.

Die Augen der Menschen und insbesonders ihre Steuerungsmechanismen sind verschieden. Daher muss für jedes Auge festgelegt werden, innerhalb welchen Bereiches (Raumwinkel) und in welche Richtung (als Zentrum dieses Bereiches) die Augenstellung bzw. Fixation noch als "zentral" oder als "bevorzugt" zu gelten hat. Es kann nötig sein, diesen Bereich ebenso wie die Ausrichtung im Laufe des Verfahrens dynamisch anzupassen.

Weil unterschiedliche Sehaufgaben in Ferne, Raumentfernungen, Greifbereich und Nähe auch unterschiedliche Blickhöhen erfordern, also gerade nach vorne in "Nullblickrichtung" bis stark nach unten beim Gehen oder Lesen, ist eine "Hauptblickrichtung" individuell für den jeweiligen Probanden festzulegen, sodass ein Bezugspunkt vorhanden ist. Weiters muss natürlich der je nach Beobachtungsentfernung und Augenabstand notwendige Konvergenzwinkel des Augenpaares berücksichtigt werden.

Die individuelle Festlegung der Blickrichtung in Relation zum Objekt im Sinne der Erfindung, die Größe des Raumwinkels des Bereiches, ab dem diese "gilt" und der Nullblickrichtung kann von Hand durch den Prüfer erfolgen, bei Verwendung einer entsprechend gesteuerten Anlage auch automatisch durch das System. Natürlich kann es notwendig werden, dass im Laufe des Verfahrens diese Werte angepasst werden müssen. Auch dies kann automatisch erfolgen, was insbesonders bei der Beachtung des Konvergenzwinkels für unterschiedliche Entfernungen notwendig ist und bei automatischer Nachführung die Arbeit vereinfacht.

Die Aufgabe der Probanden besteht nur darin, auf die angebotenen Sehobjekte zu blicken, diese zu verfolgen (wie in einem Video) oder Wörter ebenso wie einen Text vorzulesen. Es wird gleichzeitig der Status jedes einzelnen Auges sowie auch die minimalsten Änderungen der Fixation registriert, während im Laufe des Testverfahrens, in Anlehnung an alltägliche Sehaufgaben, die verschiedenen Parameter verändert werden. Als Erstes sind die Darstellungsbedingungen für Sehobjekte zu nennen, welche Schobjekte, in welcher Entfernung und unter welchen Lichtbedingung der Proband diese präsentiert bekommt. Zum Zweiten sind die Korrektionsbedingungen festzuhalten, also mit welcher insbesonders prismatischer Korrektion der Proband diese Objekte betrachtet. Zum Dritten ist die Zeit als Faktor wichtig, um zu erheben, wie lange ein Proband eine bestimmte Sehaufgabe mit einer definierten Qualität oder Schwierigkeit bewältigt bzw. wie sich diese im Laufe des Verfahrens verändert (Belastungstest).

Im Einzelnen: zu den Parametern zählt die Wahl der Entfernung. Außer der klassischen "Ferne" (≥ 5m) mit der typischen Nullblickrichtung gilt es auch, eine mittlere Entfernung von 2-3m mit dem Blick zum Boden als Äquivalent zum Gehen anzubieten. Als nächstes wird häufig der Blick auf eine mittlere Nähe von 50-70cm zur Betrachtung eines PC-Monitors bzw. zur Kontrolle der Hände (40-50cm mit tieferer Blickrichtung) zu realisieren sein. Nicht zuletzt gehört dazu das Lesen in der für die jeweilige Person typischen Entfernung und Haltung.

Zu beachten ist auch die Lichtsituation, die der jeweiligen Sehaufgabe im Alltag entsprechen soll. Dazu gehört die Simulation geringer Leuchtdichten, vorübergehend auch mit Blendung, entsprechend einer KFZ-Fahrt bei Nacht ebenso wie jene mittlerer Gesichtsfeldleuchtdichten, wie man sie in Innenräumen bei Tageslicht oder abends bei künstlicher Beleuchtung vorfindet, bis zur fotometrischen Simulation von Tageslicht im Freien.

Die Fixation und ihre Abweichung unter binokularen Bedingungen ist auch abhängig von der Art der Sehobjekte. Es können klassische Sehzeichen ebenso präsentiert werden wie geometrische Formen in positiver und negativer Darstellung oder als reine Potentialänderung vor Neutralgrau, Gittermuster in verschiedensten Ausführungen (linear; tangential, radial), alles in hohem, mittlerem wie im geringsten noch wahrnehmbaren Kontrast. Ebenso kann die Farbe des Objektes wie des Hintergrundes variieren.

Die Objekte können eine Bewegung in sich darstellen, indem sie dynamisch ihre Größe, Form, den Kontrast, die Farbe usw. wechseln oder zwischen positiver und negativer Darstellung pulsieren. Eine gute Animation zur Fixation ist auch ein Video mit beispielsweise einem Strichmännchen oder Piktogramm. Dazu kann das Objekt seinen Ort auf dem Monitor verändern und damit eine natürliche Folgebewegung bei kleinem Winkel anregen, um eine dem Alltag nicht entsprechende Fixation auf ein- und denselben Punkt über längere Zeit zu vermeiden. Allerdings dient dies, wie bereits weiter oben erwähnt, ausdrücklich nicht einer Motilitätsprüfung, also den monokularen oder binokularen Augenfolgebewegungen bei ruhig gehaltenem Kopf und stärkeren Auslenkungen in die 8 Hauptrichtungen, und ebenso ist damit keine (periphere) Gesichtsfeldmessung im Sinne einer Perimetrie verbunden, denn bevor eine Messung der Winkel und Darstellung in Relation zum Objekt erfolgen kann, muss es subjektiv wahrgenommen und fixiert werden.

Durch Verwendung von Trennvorrichtungen oder eines sogenannten "Synoptophors" können auch unterschiedliche Objekte ins rechte und linke Auge abgebildet werden (ohne Fusionanreiz) oder solche im Verein mit einem für beide Augen gleichen Objekt (mit Fusionanreiz) oder stereoskopische Bilder dargestellt werden, die nur als dreidimensionale Hervorhebung oder Vertiefung wahrnehmbar sind. Dies ist mit allen weiter oben genannten Darstellungsvariationen kombinierbar.

Während durch die Erfindung die Stellung beider Augen gemessen, gespeichert und auf dem Kontrollmonitor dargestellt werden, kann auch die subjektive Wahrnehmung durch den Probanden abgefragt werden, wie etwa bei der MKH (Mess- und Korrektionsmethodik nach H.-J. Haase). Die Erfindung ermöglicht auf dem Kontrollmonitor die Identifikation des jeweiligen Führungsauges, die rasche Entdeckung motorischer Anteile samt exakter Messung in Stärke und Richtung sowie die objektive Darstellung der Dynamik der kompensatorischen Vorgänge. Da bekanntlich eine Differenz der objektiven Abweichungen von den subjektiv angegebenen besteht, interessiert vor allem die Korrelation mit reduzierter Stereotiefe, Sehstress und verminderter Belastungsdauer.

Die Erfindung kann an und für sich als objektives Verfahren eingesetzt und, weil die Abläufe aufgezeichnet werden, auch später oder extern interpretiert werden. Als weitere Anwendung sei erwähnt, die gemessenen Stellungsabweichungen zwischen monokularen und binokularen Sehaufgaben samt ihrer kompensatorischen Dynamik, die Aufzeichnung und ihre Darstellung in Relation zu den Sehobjekten auf einem Kontrollmonitor zu kombinieren mit (subjektiven) Verfahren zur Ermittlung des besten binokularen Sehens, des Korrektionsbedarfes inklusive Prisma, sodass sich wesentlich schneller zielorientierte Entscheidungen treffen und Maßnahmen ergreifen und kontrollieren lassen, die zu einem je nach Situation besten, belastungsfreien Sehen führen.

Bekannt ist die komplexe Wechselwirkung der Augenstellung mit der Einstellrefraktion je nach Korrektion des Augenpaares - diese ist eine genau zu beachtende veränderliche Parametergruppe. Mit der Erfindung können nun erstmals, bei alltagsentsprechenden Sehaufgaben, die minimalen, für das freie Beobachterauge nicht wahrnehmbaren, kompensatorischen Reaktionen auf verschiedene Korrektionen exakt gemessen und verglichen werden, um einerseits den Bedarf einer solchen überhaupt festzustellen und, wenn sie nötig ist, die optimale Korrektion zügig zu ermitteln. Durch abschließende Tests kann dadurch für Prüfer und Proband Sicherheit geschaffen werden, dass die ermittelten Werte samt Entscheidungsfindung "richtig" sind - eine wesentliche Verbesserung gegenüber der bisherigen Situation.

Mit der Erfindung wird auch die Zeit als wichtiger Faktor in ihrer Bedeutung exakt darstellbar. Denn alle motorisch-kompensatorischen Vorgänge sind physisch belastend und können nur über eine gewisse Zeit in einer bestimmten Qualität erbracht werden. Besteht hier ein großer Aufwand und/oder eine geringe Belastbarkeit führt dies - außer zu einem unnatürlich schnellen Abfall in der Güte und Qualität des Sehens (Schschärfe, Kontrastleistung, Blendung und insbesonders Stereopsis) - zu individuellen Überlastungssymptomen, fachlich bezeichnet als "asthenopische Beschwerden" (Blendempfindlichkeit, instabile Sehschärfe, rasche Ermüdung, Konzentrationsschwäche, Kopfschmerz, "trockenes Auge", Doppelbilder).

Es ist also einerseits aufzuzeichnen, wie lange ein Proband eine Augenstellung (z.B. in Form einer exakten, zentralen Fixation beider Augen) in Relation zu einer bestimmten Sehaufgabe aufrecht halten kann. Andererseits ist, bei längerer Durchführung des Testverfahrens im Sinne eines Belastungstests, zu ermitteln, ob jemand eine "normale" Leistungsminderung aufweist oder außerhalb des üblichen Zeitrahmens (viel) früher dekompensiert, was die Betroffenen normalerweise nicht bemerken. Dies kann einerseits die Entscheidung erleichtern, eine Korrektion zu wählen, wenn dadurch die Belastbarkeit und Leistungstähigkeit des visuellen Systems signifikant, vielleicht sogar auf das allgemein übliche Niveau, gehoben wird. Andererseits sollten bestimmte Berufe oder Tätigkeiten von nur gering belastbaren Personen nicht oder nur mit (zeitlichen) Einschränkungen ausgeübt werden (Berufsberatung). Zum Dritten können alltagsentsprechend-sinnvolle Klassifikationen über Leistungs- und Belastbarkeit für Tätigkeitsberechtigungen oder mit sozialrechtlichem Aspekt geschaffen werden.

## Patentansprüche

1. Verfahren zur objektiven Messung, Aufzeichnung und Darstellung der Fixation eines Augenpaares, **gekennzeichnet dadurch, dass** nach Herstellung einer Beziehung zwischen dem visuellen Fixationsobjekt und der Augenstellung unter monokularen Bedingungen die Veränderung dieser Stellung und Bewegung unter binokularen Bedingungen gleichzeitig für beide Augen ermittelt wird.

2. Verfahren nach Anspruch 1, **dadurch gekennzeichnet, dass** die ermittelten Werte auf einem Kontrollmonitor für den Prüfer simultan dargestellt werden, indem beispielsweise sowohl das Fixationsobjekt des Probanden darauf sichtbar ist als auch, durch entsprechende unterscheidbare Symbole repräsentiert, die Fixationen beider Augen samt deren Abweichungen in Relation zu dem Fixationsobjekt.

3. Verfahren nach den Ansprüchen 1 und 2, **dadurch gekennzeichnet, dass** sowohl Richtung und Größe der Winkelabweichungen der Augen in Relation zur ursprünglichen Fixation, als auch Richtung und Winkelgröße der Blickrichtung im Verhältnis zur individuell festzulegenden "Normalblickrichtung" oder "Nullblickrichtung" gemessen, aufgezeichnet und auf einem Kontrollmonitor dargestellt werden.

4. Verfahren nach den Ansprüchen 1 bis 3, **dadurch gekennzeichnet, dass** im Verfahren die Größe des Bereiches festgelegt wird, innerhalb dessen eine Fixation als "zentral" oder "bevorzugt" (in der Peripherie) für das jeweilige Auge klassifiziert wird, wobei für das jeweilige Auge allenfalls unterschiedliche Orte und Größen erhoben werden sowie, wenn vom Probanden auf einem Auge mehrere "bevorzugte Netzhautareale" (PRL) verwendet werden, diese einzeln in Ort und Größe festzulegen, zu bezeichnen und die Messwerte entsprechend zuzuordnen sind.

5. Verfahren nach den Ansprüchen 1 bis 4, **dadurch gekennzeichnet, dass** die Festlegung einer "Hauptblickrichtung" oder "Nullblickrichtung", weiters die Größe der Bereiche, innerhalb derer eine Fixation als "zentral" oder "bevorzugt" zu bewerten ist, automatisch vom PCgestützten System vorgeschlagen und bei Bedarf im Laufe des Verfahrens auch automatisch nachgestellt wird.

6. Verfahren nach den Ansprüchen 1 bis 5, **dadurch gekennzeichnet, dass** als Anreiz zur Fixation nicht nur singuläre Objekte (wie Punkte, Optotypen, Buchstaben und Ziffern, geometrische Figuren, Piktogramme), sondern auch zusammengesetzte Objekte, einfache und komplexere Bilder bis zu Gesichtern, Wörtern und ganzen Texten verwendet werden.

7. Verfahren nach den Ansprüchen 1 bis 6, **dadurch gekennzeichnet, dass** bei Verwendung geeigneter Darstellungsmittel das ortsfeste Fixationsobjekt eine Bewegung in sich, etwa durch Änderung in Größe, Form, Kontrast, Farbe oder Kantengestaltung, Wechsel zwischen positiver und negativer Darstellung oder eine Bewegung wie in einem Zeichentrickfilm vollführt.

8. Verfahren nach den Ansprüchen 1 bis 7, **dadurch gekennzeichnet, dass** sich Fixationsobjekte unter Verwendung geeigneter Darstellungsmittel im Raum bewegen und diese Ortsveränderungen in kleinen, dem natürlichen Sehen entsprechenden Winkelabständen oder -geschwindigkeiten als Anreiz zu minimalen Augenbewegungen geschieht, ohne dass dies von der Größe her einer Motilitätsprüfung oder von der Sehaufgabe her einer Perimetrie entspricht.

9. Verfahren nach den Ansprüchen 1 bis 8, **dadurch gekennzeichnet, dass** unter Verwendung gängiger Trennverfahren unterschiedliche Objekte als Fixationsobjekte ins rechte und linke Auge abgebildet bzw. projiziert werden, wobei diese teilweise ein für das rechte und linke Auge gleiches Element als Fusionsanreiz haben können oder, um die stereoskopische Wahrnehmung zu initiieren, die Darstellung für das rechte Auge gegenüber der für das linke Auge eine gering horizontal verschobene Position aufweist.

10. Verfahren nach den Ansprüchen 1 bis 9, **dadurch gekennzeichnet, dass** sich die Fixationsobjekte bzw. ihre Darstellung in verschiedenen Entfernungen von Ferne bis Nähe und Positionshöhen befinden, die den alltäglichen Sehaufgaben oder dem Anforderungsprofil einer bestimmten Tätigkeit entsprechen.

11. Verfahren nach den Ansprüchen 1 bis 10, **dadurch gekennzeichnet, dass** die Oberflächen des Raumes, auf die der Proband blickt, das Testfeld, auf dem die visuellen Objekte dargestellt werden, sowie die Fixationsobjekte selbst Leuchtdichten aufweisen, die alltägliche Lichtsituationen entsprechen oder das Anforderungsprofil bei einer bestimmten Tätigkeit repräsentieren, wobei diese gleichmäßig oder extrem belastend sein kann, beispielsweise bei einem raschen Wechsel von Leuchtdichten oder Simulation einer Blendung.

12. Verfahren nach den Ansprüchen 1 bis 11, **dadurch gekennzeichnet, dass** die Generierung der Abbildungen neben der üblichen Präsentation im Raum durch Projektion ins Auge, durch diffraktive Systeme bzw. Interferenz oder aber durch eine Kombination der dargelegten Generierungstechniken erfolgt.

13. Verfahren nach den Ansprüchen 1 bis 12, **dadurch gekennzeichnet, dass** die motorische Reaktion bei verschiedenen refraktiven Situationen der Probanden, die insbesonders durch prismatische Korrektionen hervorgerufen werden, erhoben und miteinander verglichen werden.

14. Verfahren nach den Ansprüchen 1 bis 13, **dadurch gekennzeichnet, dass** unter Verwendung geeigneter Anordnungen bei den einzelnen Sehaufgaben exakt die Zeit ermittelt und zur Auswertung gespeichert wird, ab wann oder bis zu welchem Zeitpunkt eine (binokulare!) Fixation sich in bestimmten Bereichen oder Orten bewegt oder befunden hat.

15. Verfahren nach den Ansprüchen 1 bis 14, **dadurch gekennzeichnet, dass** unter Verwendung geeigneter Anordnungen eine Veränderung der relevanten Messgrößen sowie der Zeitpunkte und -dauer während eines länger andauernden Messverfahrens erhoben und aufgezeichnet wird, die Rückschlüsse auf den TCompensationsaufwand, die Belastbarkeit und eine (dauerhafte) Qualität des Binokularsehens erlauben.
